# EUROPEAN PATENT SPECIFICATION

(11) **EP 1 023 088 B1**
(45) Date of publication and mention of the grant of the patent: **18.02.2004**
(21) Application number: 97935107.9
(22) Date of filing: 24.07.1997
(51) Int. Cl.: A61L 15/46

(54) **PROCESS FOR MAKING PERFUME-IMPREGNATED HYDROGEL-FORMING ABSORBENT POLYMERS**
VERFAHREN ZUR HERSTELLUNG VON PARFUM-IMPRÄGNIERTEN HYDROGELE-BILDENDEN POLYMEREN
PROCEDE DE FABRICATION DE POLYMERES ABSORBANTS FORMANT UN HYDROGEL IMPREGNE DE PARFUM

(43) Date of publication of application: 02.08.2000
(73) Proprietor: THE PROCTER & GAMBLE COMPANY, Cincinnati, Ohio 45202 (US)
(72) Inventor: HSUEH, Kesyn, Fugger, Higashinada-ku, Kobe 658 (JP); REZAI, Ebrahim, Higashinada-ku, Kobe 658 (JP); RODRIGUEZ, Pedro, Antonio, Cincinnati, OH 45240 (US); MAENO, Shuji, Kita-ku, Kobe 651-11 (JP); SHIMIZU, Motohiro, Yasu-gun, Shiga 520-23 (JP)
(74) Representative: Hirsch, Uwe Thomas
(86) International application number: PCT/US1997/013014
(87) International publication number: WO 1999/004830

(56) References cited:
- EP-A- 0 328 145
- EP-A- 0 392 608
- EP-A- 0 739 635
- US-A- 4 277 582

## Description

### FIELD

The present invention relates to a process for making perfume-impregnated hydrogel-forming absorbent polymers, such polymers and disposable absorbent articles containing such polymers.

### BACKGROUND

A wide variety of disposable absorbent articles designed not only to be efficient in the absorption of body fluids such as urine, blood, menses and the like, but also, to be sanitary and comfortable in use, are known in the literature. Disposable absorbent products of this type generally comprise a fluid-permeable topsheet material, an absorbent core, and a fluid-impermeable backsheet material. Various shapes, sizes and thicknesses of such articles have been explored in an attempt to make their use more comfortable and convenient.

For some time now, studies for such disposable absorbent articles have been primarily focused on the absorptive capacity of the article. As a result, various absorbent polymers with high absorptive power have been developed. Such known absorbent polymers (also known as hydrogel-forming absorbent polymers) are capable of absorbing from about thirty to sixty grams of water per gram of polymer.

More recently, research has been focused on the removal of foul odors caused by wearing a disposable absorbent article for a relatively long time. Many body fluids have an unpleasant odor, or develop such odors when in contact with air and/or bacteria for prolonged periods. Japanese Patent Laid-open No. 61-73664 and PCT publication No. WO 9,422,500 disclose a perfume-impregnated hydrogel-forming absorbent polymers wherein the release of the perfume smell is triggered by the swelling force when the polymer is wetted, and processes for perfume-impregnating the hydrogel-forming absorbent polymer. Japanese Laid-open No. 61-73664 discloses that polymerization of the perfume-impregnated hydrogel-forming absorbent polymer is conducted by using water emulsion of perfume as a solvent. PCT publication No. WO 9,422,500 discloses that the complex of cyclodextrin and perfume is sprinkled, mixed or distributed onto the fluid absorbent material. However, a perfume-impregnated hydrogel-forming absorbent polymer wherein the release of the perfume smell is triggered by the dynamic swelling force of a wetted polymer which is made by a conventional preparing method still leaks a noticeable perfume smell even when the polymer is dry. This is undesirable because a noticeable perfume in the dry disposable article is disliked by many consumers.

Consequently, there is a need for a perfume-impregnated hydrogel-forming absorbent polymer which leaks either no perfume smell or only a negligible amount of perfume smell, (which is accepted by consumers) when the polymer is dry, and wherein the release of the perfume smell is triggered by the dynamic swelling force of the polymer when wetted, and methods of preparing the same. None of the existing art provides all of the advantages and benefits of the present invention.

### SUMMARY

The present invention is directed to a process for making a perfume-impregnated hydrogel-forming absorbent polymer (P-HFAP) wherein release of a perfume smell is triggered by the dynamic swelling force of the polymer when the polymer is wetted, which comprises the steps of (i) mixing a solid carrier and a perfume with reaction intermediates of hydrogel-forming absorbent polymer (HEAP) before the gelation point of polymerization of the hydrogel-forming absorbent polymer (HEAP), (ii) forming perfume-impregnated hydrogel-forming absorbent polymer (P-HFAP), and (iii) drying the perfume-impregnated hydrogel-forming absorbent polymer (P-HFAP).

The present invention further relates to perfume-impregnated hydrogel-forming absorbent polymer (P-HFAP) wherein the release of the perfume smell is triggered by the dynamic swelling force of the polymer when the polymer is wetted which is made by such a process.

The present invention further relates to disposable absorbent articles comprising such a perfume-impregnated hydrogel-forming absorbent polymer.

### BRIEF DESCRIPTION OF THE DRAWINGS

While the specification concludes with claims which particularly point out and distinctly claim the invention, it is believed the present invention will be better understood from the following description of preferred embodiments taken in conjunction with the accompanying drawings, in which like reference numerals identify identical elements and wherein:
Figure 1 is a plan view of a disposable diaper embodiment of the present invention, having portions cut away to reveal underlying structure, the inner surface of the diaper facing the viewer,
Figure 2 is a cross-sectional view of one embodiment of the disposable diaper of Figure 1, taken along transverse center line 110 of Figure 1;
Figure 3 is an enlarged view of an alternative embodiment, of the disposable diaper of Figure 1, the view corresponding to a portion of Figure 2; and
Figure 4 shows the key elements of the finished product acquisition test

These and other features, aspects, and advantages of the present invention will become better understood with reference to the following description and appended claims.

### DETAILED DESCRIPTION

The following is a partial list of definitions of terms used herein.

"HFAP" means hydrogel-forming absorbent polymer.

"P-HFAP" means perfume-impregnated hydrogen-forming absorbent polymer.

"Body fluids" includes urine, menses and vaginal discharges.

"Comprising" means that other steps and other ingredients which do not affect the end result can be added. This term encompasses the terms "consisting of" and "consisting essentially of".

"Disposable" describes absorbent articles which are not intended to be laundered or otherwise restored or reused as an absorbent article (*i.e.,* they are intended to be discarded after a single use and, preferably, to be recycled, composted or otherwise disposed of in an environmentally compatible manner).

"Unitary" absorbent article refers to an absorbent article which is formed of separate parts united together to form a coordinated entity so that they do not require separate manipulative parts like a separate holder and liner.

"Solid" means solid form at the temperature of 25°C.

All percentages are by weight of total composition unless specifically stated otherwise.

All ratios are weight ratios unless specifically stated otherwise.

All cited references are incorporated herein by reference in their entireties. Citation of any reference is not an admission regarding any determination as to its availability as prior art to the claimed invention.

The present invention in its process and product aspects is described in detail as follows.

The present invention relates to a process for making a P-HFAP wherein the release of the perfume smell is triggered by the dynamic swelling force of a wetted polymer. The process comprises the steps of (i) mixing a carrier and a perfume with reaction intermediates of HFAP before the gelation point of polymerization of the HFAP, (ii) forming the P-HFAP and (iii) drying the P-HFAP.

The P-HFAP which is made by such a process satisfies the need for a P-HFAP which leaks either no perfume smell or only a negligible amount of perfume smell, (which is accepted by consumers) when the polymer is dry and wherein the release of the perfume smell is triggered by the dynamic swelling force of the P-HFAP when wetted.

### A. The step of mixing a carrier and a perfume with reaction intermediates of HFAP before the gelation point of polymerization of the HFAP

The process of the present invention comprises the step of mixing a carrier and perfume with reaction intermediates of HFAP before the gelation point of polymerization of HFAP.

### a. Solid carrier

The carriers useful in the present invention include glucose and polymers such as starch and cyclodextrin, and a porous hydrophilic matrix such as zeolite, foam beads of regenerated cellulose, foam beads of hydrophilic polyurethane and foam beads of cross-linked polyvinylalcohol. Glucose is preferable and starch is more preferable for the carrier.

If the level of the carrier is less than 0.5% by weight of HFAP, it is not enough to carry an adequate amount of perfume. If the level of the carrier is more than 15% by weight of HFAP, the carriers are loosely encapsulated by HFAP, which may result in perfume smell when the polymer is dry. That level of the carrier by weight of HFAP is typically from about 0.5% to about 15%, preferably from about 1% to about 10%.

### b. perfume

The perfumes and compositions of this invention are the conventional ones known in the art Selection of any perfume component, or amount of perfume, is based on functional and aesthetic considerations. Preferred perfume components useful in the present invention are the highly volatile, and the moderately volatile perfume ingredients, more preferably the highly volatile, low boiling perfumes.

The highly volatile, low boiling, perfume ingredients typically have boiling points of about 250°C or lower. These highly volatile perfumes are fleeting and are quickly lost as they are released. Many of the more moderately volatile perfume ingredients are also quickly lost. The moderately volatile perfume ingredients are those having boiling points of from about 250°C to about 300°C. Many of the perfume ingredients as discussed hereinafter, along with their odor characters, and their physical and chemical properties, such as boiling point and molecular weight are given in "Perfume and Flavor Chemicals (Aroma Chemicals)," Steffen Arctander, published by the author, 1969, incorporated herein by reference.

Examples of the highly volatile, low boiling, perfume ingredients are: anethole, benzaldehyde, benzyl acetate, benzyl alcohol, benzyl formate, isobornyl acetate, camphene, cis-citral(neral), citronellal, citronellol, citronellyl acetate, paracymene, decanal, dihydrolinalool, dihydromyrcenol, dimethyl phenyl carbinol, eucalyptol, geranial, geraniol, geranyl acetate, geranyl nitrile, cis-3-hexenyl acetate, hydroxycitronellal, d-limonene, linalool, linalool oxide, linalyl acetate, linalyl propionate, methyl anthranilate, alpha-methyl ionone, methyl nonyl acetaldehyde, methyl phenyl carbinyl acetate, laevo-menthyl acetate, menthone, iso-menthone, myrcene, myrcenyl acetate, myrcenol, nerol, neryl acetate, nonyl acetate, phenyl ethyl alcohol, alphapinene, beta-pinene, gamma-terpinene, alpha-terpineol, beta-terpineol, terpinyl acetate, and vertenex (para-tertiary-butyl cyclohexyl acetate). Some natural oils also contain large percentages of highly volatile perfume ingredients. For example, lavandin contains as major components: linalool: linalyl acetate; geraniol; and citronellol. Lemon oil and orange terpenes both contain about 95% of d-limonene.

Examples of moderately volatile perfume ingredients are: amyl cinnamic aldehyde, iso-amyl salicylate, beta-caryophyllene, cedrene, cinnamic alcohol, coumarin, dimethyl benzyl carbinyl acetate, ethyl vanillin, eugenol, iso-eugenol, for acetate, heliotropine, 3-cis-hexenyl salicylate, hexyl salicylate, lilial (para-tertiarybutyl-alpha-methyl hydrocinnamic aldehyde), gamma-methyl ionone, nerolidol, patchouli alcohol, phenyl hexanol, beta-selinene, trichloromethyl phenyl carbinyl acetate, triethyl citrate, vanillin, and veratraldehyde. Cedarwood terpenes are composed mainly of alpha-cedrene, beta-cedrene, and other C₁₅H₂₄ sesquiterpenes.

The species of perfume odors useful in the present invention include (1) citrus note such as bergamot oil, lemon oil, sweet orange oil, bitter orange oil, lime oil, grapefruit oil, and mandarin oil; (2) fruity note such as peach, strawberry, apple, banana, melon, pineapple, raspberry, aldehyde C₁₄ (peach lactone) and aldehyde C₁₆; (3) Aldehyde note such as C₇₋₁₂ fatty acid aldehyde; (4) floral note such as rose, jasmine, carnation, and honeysuckle; (5) spicy note such as clove, cinnamon Ceylon & China, pepper, pimento, coriander, cardamom, nutmeg, and bay; (6) woody note such as sandal wood, cedar wood, vetiver and patchouli; (7) animal note such as castoreum, ambergris, civet, and musk (preferably obtained by synthesis); (8) minty note such as peppermint and spearmint; (9) honey note such as absolute honey; (10) vanillic note such as vanilla, (11) anise note such as anise, anise star, sweet fennel, basil and tarragon; (12) lavender note such as lavender oil, lavandin oil, rosemary oil and eucalyptus oil; (13) green note such as absolute violet leaves; (14) mossy note such as absolute oakmoss; (15) balsamic note such as tolu balsam, Peru balsam and benzoin; (16) coniferous note such as pine oil and juniperberry oil.

Other perfumes useful herein include (1) Baby Floral: C-5133, 5134, or 5138, supplied by Soda Aromatic CO. LTD. at Shiga, Japan, (2) Pentalide series, e.g. A-1559; Primax series, e.g., A-1561, supplied by Soda Aromatic CO. LTD. at Shiga, Japan, (3) Floramild series, e.g., N-30131, supplied by Haarman & Reimer, Germany, and (4) Floral fragrance GH-8125-8130 and FNT-849, 850, supplied by Takasago Aromatic Co. LTD. Osaka, Japan.

The amount of the perfume which is sufficient to provide efficacious perfume delivery properties can vary based on a number of factors such as the chemical composition of the HEAP and the physical forms of the HFAP, e.g., particle size of the HFAP, and the chemical composition of the perfume, as well as on the nature of carrier system and the method of applying the perfume and carrier. However, typically, if the level of the perfume is less than 0.01% by weight of HFAP, the smell of perfume when the polymer is wet is insufficiently strong. If the level of the perfume is more than 20% by weight of HFAP, the smell of perfume when the polymer is dry is too strong.

In preferred embodiments, the weight ratio of HFAP to perfume is from about 100:0.01 to about 100:20, more preferably from about 100:0.1 to about 100:10, more preferably still from about 100:0.5 to about 100:5.0.

Preferably, the perfume is preliminarily included in the carrier before addition to the reaction intermediates of HFAP.

In a case where the perfume is in the form of a small particle or powder, the perfume can be mixed with a carrier by any of various techniques and apparatus used for applying (e.g., mixture) a material to another material that are known in the art. In an another case where the perfume is in the form of a liquid, the perfume can be included in a carrier by any of various techniques and apparatus used for applying a liquid to a material that are known in the art.

In a preferred embodiment, perfume is loaded into a glucose carrier to make a solid solution. The perfume can be absorbed or adsorbed in the carrier by any of various techniques and apparatus used for loading a material to a carrier known in the art. If the level of the perfume is less than 3% by weight of the Carrier, it is not efficacious. If the level of the perfume is more than 90% by weight of the carrier, the carrier is overloaded, and perfume leaks out too easily from the carrier and the HFAP. Preferably, the concentration of the perfume in the carrier by weight is from about 3% to 90%, more preferably, from about 20% to 70%.

The carrier and the perfume are applied into a material mixture of HFAP before the gelation point of HFAP polymerization at a temperature of typically from about 50°C to about 90°C, preferably from about 60°C to about 80°C under atmosphere or inert gas, preferably, from about a few seconds to about 20 minutes before the gelation point of the polymerization, more preferably from about 5 minutes to about 15 minutes before the gelation point of the polymerization.

If the carrier and perfume are applied into the material mixture of HEAP more than about 20 minutes before the gelation point of the polymerization, perfume interferes with the polymerization and the perfume is prone to evaporation. If the carrier and perfume are applied into the material mixture of HEAP less than about a few seconds before the gelation point of the polymerization, and after the gelation point of the polymerization, perfume cannot be trapped and protected in the dry state.

### c. Reaction intermediates of HFAP

The reaction intermediates of HFAP include linear polymers, oligomers monomers and a crosslinker, and may also include residual polymerization initiator and water.

The linear polymers and the oligomers are intermediates of HFAP made from the monomers as below.

The monomers include polymerizable, unsaturated, acid-containing monomers; some non-acid monomers; olefinically unsaturated carboxylic acid and carboxylic acid anhydride monomers: and olefinically unsaturated sulfonic acid monomers.

Unsaturated, acid-containing monomers include the olefinically unsaturated acids and anhydrides that contain at least one carbon to carbon olefinic double bond. More specifically, these monomers can be selected from olefinically unsaturated carboxylic acids and acid anhydrides, olefinically unsaturated sulfonic acids, and mixtures thereof.

Some non-acid monomers can also be included, usually in minor amounts, in preparing the HFAPs herein. Such non-acid monomers can include, for example, the water-soluble or water-dispersible esters of the acid-containing monomers, as well as monomers that contain no carboxylic or sulfonic acid groups at all. Optional non-acid monomers can thus include monomers containing the following types of functional groups: carboxylic acid or sulfonic acid esters, hydroxyl groups, amide-groups, amino groups, nitrile groups, quaternary ammonium salt groups, aryl groups (e.g., phenyl groups, such as those derived from styrene monomer). These non-acid monomers are well-known materials and are described in greater detail, for example, in U.S. Patent 4,076,663 (Masuda et al), issued February 28, 1978; and in U.S. Patent 4,062,817 (Westerman), issued December 13,1977.

Olefinically unsaturated carboxylic acid and carboxylic acid anhydride monomers include the acrylic acids typified by acrylic acid itself, acrylates, methacrylic acid, ethacrylic acid, 3-chloroacrylic acid, cyanoacrylic acid, methacrylic acid (crotonic acid), phenylacrylic acid, acryloloxypropionic acid, sorbic acid, chlorosorbic acid, angelic acid, cinnamic acid, p-chlorocinnamic acid, sterylacrylic acid, itaconic acid, citroaconic acid, mesaconic acid, glutaconic acid, aconitic acid, maleic acid, fumaric acid, tricarboxyethylene and maleic acid anhydride.

Olefinically unsaturated sulfonic acid monomers include aliphatic or aromatic vinyl sulfonic acids such as vinylsulfonic acid, allyl sulfonic acid, vinyl toluene sulfonic acid and styrene sulfonic acid; acrylic and methacrylic sulfonic acid such as sulfoethyl acrylate, sulfoethyl methacrylate, sulfopropyl acrylate, sulfopropyl methacrylate, 2-hydroxy-3-methacryloxypropyl sulfonic acid and 2-acrylamide-2-methylpropane sulfonic acid.

Before polymerization of HFAP starts, the level of the monomers by moles of total starting materials of HFAP is typically from about 3% to about 99.8%, preferably from about 5% to about 40%.

The crosslinker includes methylene bis acrylamide, trimethylpropane triacrylate, and polyethylene glycol diacrylate. If the level of crosslinker by moles of the total starting materials of HFAP is more than 0.1% before polymerization of HFAP starts, the swelling degree of gel in water is dramatically reduced, thereby reducing the absorptive capacity of the polymer. If the level of crosslinker by moles of the total starting materials of HFAP is less than about 0.001% before polymerization of HFAP starts, the gel is very weak and tends to dissolve when in contact with water. Before polymerization of HFAP starts, the level of crosslinker by moles of the total starting materials of HFAP is typically from about 0.001% to about 0.1%, preferably from about 0.002% to about 0.02%.

The polymerization initiator, includes sodium persulfate, hydrogen peroxide, 1-ascorbic acid, sodium thiosulfate and sodium sulfite.

If the level of the polymerization initiator by moles of the total starting materials of HFAP is more than about 0.1% before polymerization of HFAP starts, produced P-HFAP is more soluble in water, which is not preferable because the polymer, when wetted may leak out from the article. Before polymerization of HFAP starts, the level of the polymerization initiator by moles of total starting materials of HFAP is typically from 0% to about 0.1%, preferably from 0% to about 0.005%.

Before polymerization of HFAP starts, if the level of water by moles of the total starting materials of HFAP is more than 96.999, excessive drying time is required, which is not economical. Before polymerization of HFAP starts the level of water by moles of the total starting materials of HFAP is typically from 0% to about 96.999%, preferably about 59.975% to about 94.998%.

The carrier and the perfume can be applied to the reaction intermediates of the HFAP by any of various techniques and apparatus used for applying carrier to a slurry material known in the art including agitating, atomizing and dispersing, or embodying a solid mixture into a forming absorbent gelling system.

### d. Hydrogel-Forming Absorbent Polymer

The water-insoluble, water-swellable absorbent polymers useful for being impregnated with perfume in the present invention are commonly referred to as "hydrogel-forming absorbent polymers" (HFAPs), "hydrocolloids", or "superabsorbent" polymers and can include polysaccharides such as carboxymethyl starch, carboxymethyl cellulose, and hydroxypropyl cellulose; nonionic types such as polyvinyl alcohol, and polyvinyl ethers; cationic types such as polyvinyl pyridine, polyvinyl morpholinione, and N,N-dimethylaminoethyl or N,N-diethylaminopropyl acrylates and methacrylates, and the respective quaternary salts thereof. Typically, HFAPs useful in the present invention have a multiplicity of anionic, functional groups, such as sulfonic acid, and preferably carboxy, groups.

Preferred HFAPs for use in the present invention contain carboxy groups. These polymers include hydrolyzed starch-acrylonitrile graft copolymers, partial neutralized hydrolyzed starch-acrylonitrile graft copolymers, starch-acrylic acid graft copolymers, partially neutralized starch-acrylic acid graft copolymers, saponified vinyl acetate-acrylic ester copolymers, hydrolyzed acrylonitrile or acrylamide copolymers, slightly network crosslinked polymers of any of the foregoing copolymers, partially neutralized polyacrylic acid, and slightly network crosslinked polymers of partially neutralized polyacrylic acid. These polymers can be used either solely or in the form of a mixture of two or more different polymers. Examples of these polymer materials are disclosed in U.S. Patent 3,661,875, U.S. Patent 4,076,663, U.S. Patent 4,093,776, U.S. Patent 4,666,983, and U.S. Patent 4,734,478.

More preferred polymer materials for use in making the HFAPs are slightly-networked, crosslinked polymers of partially neutralized polyacrylic acids and starch derivatives thereof. More preferably, these HFAPs comprise from about 50 to about 95%, preferably about 75%, neutralized, slightly-networked, crosslinked, polyacrylic acid (*i.e.*, poly (sodium acrylate/acrylic acid)). Network crosslinking renders the polymer substantially water-insoluble and, in part, determines the absorptive capacity and extractable polymer content characteristics of the HFAPs. Processes for network crosslinking these polymers and typical network crosslinking agents are described in U.S. Patent 4,076,663.

While the HEAP is preferably of one type *(i.e.,* homogeneous), mixtures of polymers can also be used in the present invention. For example, mixtures of starch-acrylic acid graft copolymers and slightly-networked, crosslinked polymers of partially neutralized polyacrylic acid can be used in the present invention.

The HEAP particles used in the present invention can have a size, shape. and/or morphology varying over a wide range. (*e.g.*, granules. flakes, pulverulents, interparticle aggregates, interparticle crosslinked aggregates, and the like) and can be in the form of fibers, foams, and the like. The HFAPs can also comprise, mixtures with low levels of one or more additives, such as for example powdered silica, surfactants, glue, binders, and the like. The components in this mixture can be physically and/or chemically associated in a form such that the hydrogel-forming polymer component and the non-hydrogel-forming polymer additive are not readily physically separable.

The HFAPs can be essentially non-porous or have substantial internal porosity.

For particles as described above, particle size is defined as the dimension determined by sieve size analysis. Thus, for example, a particle that is retained on a U.S.A. Standard Testing Sieve with 710 micron openings (*e.g.*, No. 25 U.S. Series Alternate Sieve Designation) is considered to have a size greater than 710 microns; a particle that passes through a sieve with 710 micron openings and is retained on a sieve with 500 micron openings (*e.g.*, No. 35 U.S, Series Alternate Sieve Designation) is considered to have a particle size between 500 and 710 microns; and a particle that passes through a sieve with 500 micron openings is considered to have a size less than 500 microns. The mass median particle size of a given sample of hydrogel-forming absorbent polymer particles is defined as the particle size that divides the sample in half on a mass basis, *i.e.,* one-half of the sample by weight will have a particle size less than the mass median size and one-half of the sample will have a particle size greater than the mass median size. A standard parade-size plotting method (wherein the cumulative weight percent of the particle sample retained on or passed through a given sieve size opening is plotted versus sieve size opening on probability paper) is typically used to determine mass median particle size when the 50% mass value does not correspond to the size opening of a U.SA. Standard Testing Sieve. These methods for determining particle sizes of the hydrogel-forming absorbent polymer particles are further described in U.S. Patent 5,061,259 (Goldman *et. al*), issued October 29, 1991.

For particles of HFAPs useful in the present invention, the particle size will generally range from about 1 to about 2000 microns, more preferably from about 20 to about 1000 microns. The mass median particle size will generally be from about 20 to about 1500 microns, more preferably from about 50 microns to about 1000 microns, and even more preferably from about 100 to about 800 microns.

Within these size ranges, it can be preferable to choose either larger or smaller particles depending on the need for faster or slower absorption kinetics. For example, for non-porous particles, the swelling rate will generally decrease with increasing particle size. It can also be preferable to choose either larger or smaller particles or narrower size cuts (fractions) of larger or smaller particles from the bulk polymer in order to increase the gel layer permeability (i.e., increase the Saline Flow Conductivity (SFC) value). For particles of some HFAPs, it has been found that narrower size range cuts containing, generally larger particle sizes within the above specified size ranges have higher SFC values without any significant degradation in other HFAP properties such as Performance Under Pressure (PUP) capacity and level of extractable polymer. Thus, for example, it can be useful to use a size cut having a mass median size in the range of from about 500 to about 710 microns wherein only minimal mass fractions of the particulates have sizes either greater than about 710 microns or less than about 500 microns. Alternatively, a broader size cut wherein the particles generally have a size in the range of from about 150 microns to about 800 microns can be useful.

### B. The step of forming P-HFAP

In from about few seconds to about 20 minutes after mixing the carrier and the perfume with the reaction intermediates of HFAP before the gelation point of polymerization of HFAP, gelation starts as indicated by elevation of the temperature by 5 - 10°C automatically. Gelation is when polymer starts forming a network, becoming virtually insoluble in any solvent.

After the gelation starts, the temperature of the reaction intermediates is kept at from about 60°C to about 90°C for another about 0.5 hours to about 2 hours in order to complete the gelation.

Thus, in the mixing of a carrier and a perfume with reaction intermediates of HFAP before the gelation point of polymerization of the HFAP, the perfume and carrier will be impregnated in at least a portion of the interior of the HFAP to form P-HFAP. Preferably, the perfume and carrier will be in all of the interior of the HFAP particles.

### C. The step of drying the P-HFAP

After forming P-HFAP, the water solvent for HFAP is removed from the format P-HFAP. Preferably, at least about 30%, more preferably more than 50% of the water solvent is removed from the intermittent mixture. The removal of the solvent can be made by any of various techniques and apparatus used for separating or removing liquids from liquid-solid mixture known in the art, including evaporation, filtration, or a combination thereof.

### E. Absorbent Articles Employing the Perfume-impregnated Hydrogel-Forming Absorbent Polymer

The P-HFAPs according to the present invention can be used for many purposes in many fields of use. For example, the P-HFAPs can be used for packing containers; drug delivery devices; wound cleaning devices; burn treatment devices; ion exchange column materials; construction materials; agricultural or horticultural materials such as seed sheets or water-retentive materials; and industrial uses such as sludge or oil dewatering agents, materials for the prevention of dew formation, desiccants, and humidity control materials.

Because of the unique absorbent and perfume delivery properties of the P-HFAPs of the present invention, they are especially suitable for use as absorbent cores in absorbent articles, especially disposable absorbent articles. As used herein, the term "absorbent article" refers to articles which absorb and contain body fluids and more specifically refers to articles which are placed against or in proximity to the body of the wearer to absorb and contain the various fluids discharged from the body.

In general, an absorbent article comprises: (a) a liquid pervious topsheet which is located adjacent to the wearer's body; (b) a liquid impervious backsheet which is located distant from the wearer's body and adjacent to the wearer's clothing; and (c) an absorbent core positioned between the topsheet and the backsheet. The absorbent core comprises at least one of the above described P-HFAPs of the present invention. Preferably, the absorbent core further comprises a substrate web wherein the absorbent material is attached to the substrate web. Alternatively, the absorbent core further comprises an envelope web encasing the absorbent material. In a further alternative embodiment, the absorbent core further comprises two layered tissues wherein the absorbent material is distributed between the two layered tissues.

In more preferred embodiments, the P-HFAP in the absorbent core has a basis weight of from about 60 g/m² to about 1500 g/m², more preferably from about 100 g/m² to about 1000 g/m², most preferably from about 150 g/m² to about 500 g/m² of the absorbent material.

In some preferred embodiments, the absorbent core or absorbent member can further comprise fibers or fluff pulp (fibrous or fiber material), more specifically, non-absorbent-gelling fibers. Such fiber material can be used as reinforcing members in the absorbent core, improving fluid handling of the core, as well as a co-absorbent with the absorbent polymers. Preferably, the absorbent core or member includes from about 40% to about 100% by weight of the P-HFAP and from about 0% to about 60% by weight of such non-absorbent-gelling fiber material distributed within the absorbent material.

In a preferred embodiment, the P-HFAP is in a concentration of at least 40%, more preferably from about 60 to 100% by weight in at least one region of the core or absorbent member. In a more preferred embodiment, the absorbent member comprises fibrous matrix wherein the P-HFAP is distributed in the fibrous matrix.

Any type of fiber material which is suitable for use in conventional absorbent products can be used in the absorbent core or absorbent member herein. Specific examples of such fiber material include cellulose fibers, improved cellulose fibers, rayon, polypropylene, and polyester fibers such as polyethylene terephthalate (DACRON), hydrophilic nylon (HYDROFIL), and the like. Examples of other fiber materials for use in the present invention include hydrophilized hydrophobic fibers, such as surfactant-treated or silica-treated thermoplastic fibers derived, for example, from polyolefins such as polyethylene or polypropylene, polyacrylics, polyamides, polystyrenes, polyurethanes and the like. In fact, hydrophilized hydrophobic fibers which are in and of themselves not very absorbent and which, therefore, do not provide webs of sufficient absorbent capacity to be useful in conventional absorbent structures, are suitable for use in the absorbent core by virtue of their good wicking properties. This is because, in the absorbent core herein, the wicking propensity of the fibers is as important, if not more important, than the absorbent capacity of the fiber material itself due to the high rate of fluid uptake and lack of gel blocking properties of the absorbent core. Synthetic fibers are generally preferred for use herein as the fiber component of the absorbent core. Most preferred are polyolefin fibers, preferably polyethylene fibers.

Other cellulosic fiber materials which can be useful in certain absorbent cores or absorbent members herein are chemically stiffened cellulosic fibers. Preferred chemically stiffened cellulosic fibers are the stiffened, twisted, curled cellulosic fibers which can be produced by internally crosslinking cellulose fibers with a crosslinking agent. Suitable stiffened, twisted, curled cellulose fibers useful as the hydrophilic fiber material herein are described in greater detail in U.S. Patent 4,888,093 (Dean et al), issued December 19, 1989; U.S., Patent 4,889,595 (Herron et al), issued December 26, 1989; U.S. Patent 4,889,596 (Schoggen et al), issued December 26, 1989; U.S. Patent 4,889,597 (Bourbon et al), issued December 26, 1989; and U.S. Patent 4,898,647 (Moore et al), issued February 6, 1990.

A preferred embodiment of the disposable absorbent article is a diaper. As used herein, the term "diaper" refers to a garment generally worn by infants and incontinent persons that is worn about the lower torso of the wearer. A preferred diaper configuration for a diaper comprising an absorbent core is described generally in U.S. Patent 3,860,003 (Buell), issued January 14, 1975. Alternatively preferred configurations for disposable diapers herein are also disclosed in U.S. Patent 4,808,178 (Aziz et al), issued February 28, 1989; U.S. Patent 4,695,278 (Lawson), issued September 22, 1987; U.S. Patent 4,816,025 (Foreman), issued March 28, 1989; and U.S. Patent 5,151,092 (Buell; et al), issued September 29, 1992.

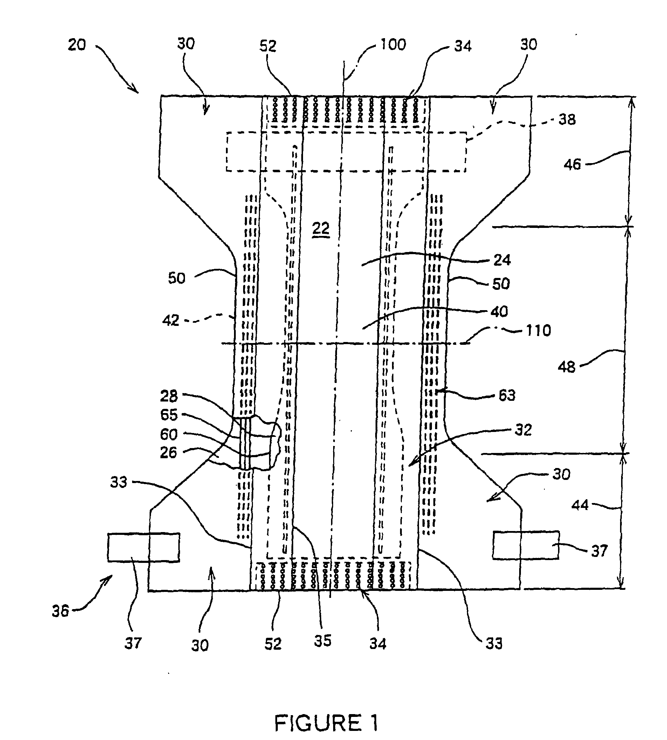

A preferred embodiment of an absorbent article of the present invention is the unitary disposable absorbent article, diaper 20, shown in Figure 1. Figure 1 is a plan view of the diaper 20 of the present invention in its flat-out, uncontracted state (*i.e.*, with elastic induced contraction pulled out) with portions of the structure being cut-away to more clearly show the construction of the diaper 20 and with the portion of the diaper 20 which faces the wearer, the inner surface 40, facing the viewer. As shown in Figure 1, the diaper 20 preferably comprises a containment assembly 22 comprising a liquid pervious topsheet 24; a liquid impervious backsheet 26 joined to the topsheet; and an absorbent core 28 positioned between the topsheet 24 and the backsheet 26. The absorbent core 28 has a pair of opposing longitudinal edges 60, an inner surface 62 and an outer surface 64. The diaper preferably further comprises side panels 30; elasticized leg cuffs 32; elasticized waistbands 34; and a fastening system 36 preferably comprising a pair of securement members 37 and a landing member 38.

The diaper 20 is shown in Figure 1 to have an inner surface 40 (facing the viewer in Figure 1), an outer surface 42 opposed to the inner surface 40, a rear waist region 44, a front waist region 46 opposed to the rear waist region 44, a crotch region 48 positioned between the rear waist region 44 and the front waist region 46, and a periphery which is defined by the outer perimeter or edges of the diaper 20 in which the side edges are designated 50 and the end edges are designated 52. The inner surface 40 of the diaper 20 comprises that portion of the diaper 20 which is positioned adjacent to the wearers body during use (*i.e.,* the inner surface 40 generally is formed by at least a portion of the topsheet 24 and other components joined to the topsheet 24). The outer surface 42 comprises that portion of the diaper 20 which is positioned away from the wearer's body (i.e., the outer surface 42 is generally formed by at least a portion of the backsheet 26 and other components joined to the backsheet 26). As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. The rear waist region 44 and the front waist region 46 extend from the end edges 52 of the periphery to the crotch region 48.

The diaper 20 also has two centerlines, a longitudinal centerline 100 and a transverse centerline 110. The term "longitudinal", as used herein, refers to a line, axis, or direction in the plane of the diaper 20 that is generally aligned with (*e.g.* approximately parallel with) a vertical plane which bisects a standing wearer into left and right halves when the diaper 20 is worn. The terms "transverse" and "lateral", as used herein, are interchangeable and refer to a line, axis or direction which lies within the plane of the diaper that is generally perpendicular to the longitudinal direction (which divides the wearer into front and back body halves).

The containment assembly 22 of the diaper 20 is shown in Figure 1 as comprising the main body (chassis) of the diaper 20. The containment assembly 22 preferably comprises a topsheet 24, a backsheet 26 and an absorbent core 28 having a pair of opposing longitudinal edges 60, an inner surface 62, an outer surface 64. The inner surface 62 generally faces the body of the wearer while the outer surface 64 generally faces away from the body of the wearer. When the absorbent article comprises a separate holder and a liner, the containment assembly 22 generally comprises the holder and the liner (*i.e.,* the containment assembly 22 comprises one or more layers of material to define the holder while the liner comprises an absorbent composite such as a topsheet, a backsheet, and an absorbent core.) For unitary absorbent articles, the containment assembly 22 preferably comprises the topsheet 24, the backsheet 26 and the absorbent core 28 of the diaper with other features added to form the composite diaper structure.

Figure 1 shows a preferred embodiment of the containment assembly 22 in which the topsheet 24 and the backsheet 26 have length and width dimensions generally larger than those of the absorbent core 28. The topsheet 24 and the backsheet 26 extend beyond the edges of the absorbent core 28 to thereby form the periphery of the diaper 20. While the topsheet 24, the backsheet 26, and the absorbent core 28 may be assembled in a variety of weft known configurations, exemplary containment assembly configurations are described generally in U.S. Patent 3,860,003 entitled "Contractible Side Portions for Disposable Diaper" which issued to Kenneth B. Buell on January 14, 1975; and U.S., Patent 5,151,092 entitled "Absorbent Article With Dynamic Elastic Waist Feature Having A Predisposed Resilient Flexural Hinge" which issued to Kenneth B. Buell et al., on September 29, 1992; each of which is incorporated herein by reference.

The absorbent core 28 may be any absorbent member which is generally compressible, conformable, non-irritating to the wearer's skin, and capable of absorbing and retaining liquids such as urine and other certain body exudates. As shown in Figure 1, the absorbent core 28 has a garment-facing side, a body-facing side, a pair of side edges, and a pair of waist edges. The absorbent core 28 may be manufactured in a wide variety of sizes and shapes (*e.g.*, rectangular, hourglass, 'T'-shaped, asymmetric, etc.) and, in addition to including an P-HFAP of the present invention, may also include a wide variety of liquid-absorbent materials commonly used in disposable diapers and other absorbent articles such as comminuted wood pulp which is generally referred to as airfell. Examples of other suitable absorbent materials include creped cellulose wadding; mettblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 28 may vary (*e.g.*, the absorbent core may have varying caliper zones, a hydrophilic gradient a superabsorbent gradient, or lower average density and lower average basis weight acquisition zones; or may comprise one or more layers or structures). Further, the size and absorbent capacity of the absorbent core 28 may also be varied to accommodate wearers ranging from infants through adults. However, the total absorbent capacity of the absorbent core 28 should be compatible with the design loading and the intended use of the diaper 20.

One embodiment of the diaper 20 has an asymmetric, modified T-shaped absorbent core 28 having ears in the front waist region but a generally rectangular shape in the rear waist region. Exemplary absorbent structures for use as the absorbent core 28 of the present invention that have achieved wide acceptance and commercial success are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989. The absorbent core may further comprise the dual core system containing an acquisition/distribution core of chemically stiffened fibers positioned over an absorbent storage core as detailed in U.S. Patent 5,234,423, entitled "Absorbent Article With Elastic Waist Feature and Enhanced Absorbency" issued to Alemany et al., on August 10. 1993; and in U.S. Patent 5,147,345, entitled "High Efficiency Absorbent Articles For Incontinence Management" issued to Young, LaVon and Taylor on September 15, 1992.

The topsheet 24 is preferably positioned adjacent the inner surface 62 of the absorbent core 28 and is preferably joined thereto and to the backsheet 26 by attachment means (not shown) such as those well known in the art. Suitable attachment means are described with respect to joining the backsheet 26 to the absorbent core 28. In a preferred embodiment of the present invention, the topsheet 24 and the backsheet 26 are joined directly to each other in the diaper periphery and are indirectly joined together by directly joining them to the absorbent core 28 by any suitable attachment means.

The topsheet 24 is preferably compliant, soft feeling, and non-irritating to the wearer's skin. Further, the topsheet 24 is preferably liquid pervious permitting liquids (*e.g.*, urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (*e.g.*, wood or cotton fibers), synthetic fibers (*e.g.*, polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 28 (*i.e.* to prevent rewet). If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 28. The topsheet 24 can be rendered hydrophilic by treating it with a surfactant. Suitable methods for treating the topsheet 24 with a surfactant include spraying the topsheet 24 material with the surfactant and immersing the material into the surfactant. A more detailed discussion of such a treatment and hydrophilicity is contained in U.S. Patents 4,988,344 entitled "Absorbent Articles with Multiple Layer Absorbent Layers" issued to Reising, et al on January 29, 1991 and U.S. Patent 4,988,345 entitled "Absorbent Articles with Rapid Acquiring Absorbent Cores" issued to Reising on January 29, 1991.

An alternative preferred topsheet comprises an apertured formed film. Apertured formed films are preferred for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent 3,929,135, entitled "Absorptive Structures Having Tapered Capillaries", which issued to Thompson on December 30, 1975; U.S. Patent 4,324, 246 entitled "Disposable Absorbent Article Having A Stain Resistant Topsheet", which issued to Mullane, et al. on April 13, 1982; U.S: Patent 4,342,314 entitled "Resilient Plastic Web Exhibiting Fiber-Like Properties which issued to Radel. et al. on August 3, 1982; U.S. Patent 4,463,045 entitled "Macroscopically Expanded Three-Dimensional Plastic Web Exhibiting Non-Glossy Visible Surface and Cloth-Like Tactile Impression", which issued to Ahr et al. on July- 31, 1984: and U.S. 5,006.394 "Multilayer Polymeric Film" issued to Baird on April 9, 1991.

The backsheet 26 of the present invention is that portion of the diaper 20 which is generally positioned away from the wearer's skin and which prevents the exudates absorbed and contained in the absorbent core 28 from wetting articles which contact the diaper 20 such as bedsheets and undergarments. Thus, the backsheet 26 is preferably impervious to liquids (e.g., urine) and is preferably manufactured from a thin plastic film, although other flexible liquid impervious materials may also be used. (As used herein, the term "flexible" refers to materials which are compliant and will readily conform to the general shape and contours of the human body.) However, the backsheet 26 permits vapors to escape from the diaper 20. A suitable material for the backsheet 26 is a thermoplastic film having a thickness of from about 0.012 mm (0.5 mil) to about 0.051 mm (2.0 mils), preferably comprising polyethylene or polypropylene.

The backsheet 26 of the present invention may comprise a single member such as the film described above, or may comprise a number of materials joined together to form the backsheet 26. For example, the backsheet may have a central region 74 comprising one film or other member and one or more outer regions 76 joined to the central region 74 comprising the same or different films or other materials. In one preferred embodiment, the backsheet 26 comprises a central region 76 comprising a liquid impervious, non-apertured film and two opposing outer regions 76 comprising an air pervious, apertured film. The means by which any portions of such a backsheet are joined may include any means known in the art such as adhesives, heat, pressure, heat and pressure and ultrasonic bonds. Further, the backsheet 26 may comprise any number of layers of material joined together to form a laminate. If the backsheet 26 is a laminate, the layers need not be uniform throughout the backsheet. For example, the central region 74 of the backsheet 26 may comprise more layers or layers of different material than the outer regions 76.

The backsheet 26 is preferably positioned adjacent the outer surface 64 of the absorbent core 28 and is preferably joined thereto by any suitable attachment means known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. An example of a suitable attachment means comprising an open pattern network of filaments of adhesive is disclosed in U.S. Patent 4,573,986 entitled "Disposable Waste-Containment Garment", which issued to Minetola et al. on March 4, 1986. Another suitable attachment means comprising several lines of adhesive filaments swirled into a spiral pattern is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents are incorporated herein by reference. Alternatively, the attachment means may comprise heat bonds, pressure bonds, ultrasonic bonds, dynamic mechanical bonds, or any other suitable attachment means or combinations of these attachment means as are known in the art.

Embodiments of the present invention are also contemplated wherein the absorbent core is not joined to the backsheet 26, and/or the topsheet 24 in order to provide greater extensibility in the front waist region 46 and the rear waist region 44. Alternative embodiments are contemplated wherein an additional member, such as a liquid impervious barrier material(s) (not shown), is positioned between the outer surface 64 of the absorbent core 28 and the backsheet 28. Any such barrier member may or may not be joined to the absorbent core 28. Further, the backsheet 26 may or may not be joined to any barrier material(s) that are positioned between the backsheet 26 and the absorbent core 28.

Another preferred embodiment of the disposable absorbent article is a catamenial product. Preferred catamenial products comprise a formed-film, apertured topsheet as disclosed in U.S. Patent 4,285,343 (McNair), issued August 25, 1981; U.S. Patent 4,608,047 (Mattingly), issued August 26, 1986; and U.S. Patent 4,687,478 (Van Tilburg), issued August 18, 1987. Preferred catamenial products can comprise wings, side flaps, and other structures and elements.

A particularly preferred disposable absorbent article comprising the P-HFAP has a reduced tendency for surface wetness. Such an article facilitates maintaining the antimicrobial away from the user's skin, even after wetting. A disposable absorbent article comprising a backsheet, a topsheet, an acquisition/distribution layer, and an absorbent core; the absorbent core comprising the P-HFAP of the present invention. Such an absorbent article preferably has a total finished product acquisition rate (test method discussed below) greater than or equal to about 1.9 ml/sec at first gush and greater than or equal to about 0.3 ml/sec at fourth gush. The folded stackheight of such an absorbent article is preferably less than about 9.9 mm per pad, more preferably less than about 6 mm. Preferably the topsheet of such a product retains no more than about 0.05 g to about 1 g of fluid as measured by the finished product wetness test (discussed below); more preferably no more than about 0.5 g of fluid; more preferably, no more than about 0.2 g of fluid; more preferably still, no more than about 0.15 g of fluid.

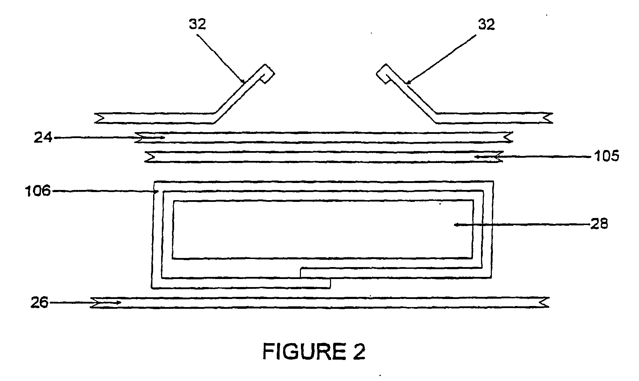

Referring to Figure 2, an exemplary embodiment of such a disposable absorbent article in the form of a diaper is provided. Figure 2 is a cross-sectional view of such an embodiment of the disposable diaper of Figure 1, taken along transverse center line 110 of Figure 1. This view shows fragmentary cross-sectional views of the backsheet 26, core cover 22, absorbent core 28, topsheet 24, and elasticized leg cuffs 32. This embodiment differs from that shown in Figure 1, in that an additional element, acquisition/distribution layer 105 has been added.

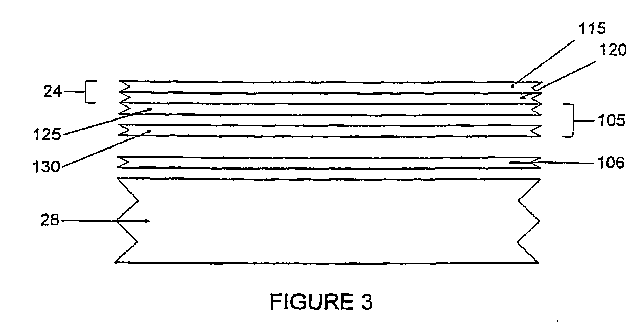

Figure 3 is an enlarged view of an alternative embodiment of the disposable diaper of Figure 1, the view corresponding to a portion of Figure 2. In this embodiment, topsheet 24 comprises first topsheet layer 115 and; second topsheet layer 120. First topsheet layer 115 and second topsheet layer 120 are preferably heat bonded together. Acquisition/distribution layer 105 comprises a first acquisition/distribution layer 125 and a second acquisition/distribution layer 130. First acquisition/distribution layer 125 is preferably spiral glue bonded to second topsheet layer 120. Preferably first acquisition/distribution layer 125 and second acquisition/distribution layer 130 are not bonded together.

Either embodiment set forth in Figure 2 or Figure 3 provides reduced tendency for surface wetness; and consequently facilitates maintaining the antimicrobial away from the user's skin, even after wetting. In other words, such an embodiment reduces the chance that liquid, *e.g*., urine having come in contact with the P-HFAP and now possibly containing antimicrobial, will flow back to the user's skin, bringing the antimicrobial in contact with the user's skin.

Preferred topsheet 24 and acquisition/distribution layer 105 materials for such a reduced surface wetness absorbent article include the following:

Preferred topsheet 24 material is P-8 nonwoven available from Fiberweb North America, Inc. (Simpsonville, South Carolina, U.S.A.). It is a conventional thermobonded carded web of about 20 to 22 g/m2. made of polypropylene fibers of about 2.2 dtex and an easily removable surfactant (spin finish), *i.e.,* at a first fluid insult, it is very hydrophilic, but at repeated wetting it is essentially as hydrophobic as the base polypropylene.

More preferably, the topsheet 24 material is S-2355, available from Havix Co., Japan. This is a bi-layer composite material, and made of two kinds of synthetic surfactant treated bicomponent fibers by using carding and air-through technologies. First topsheet layer 115 is preferably a polypropylene/polypropylene bicomponent fiber, e.g., a lower melting temperature polypropylene in sheath and a higher melting temperature polypropylene in the core of the fiber. Second topsheet layer 120 is preferably a polyethylene/polyethylene terephthalate bicomponent fiber, e.g:, a lower melting temperature polyethylene in the sheath and a higher melting temperature and more resilient polyethylene terephthalate in the core of the fiber. The first topsheet layer 115 preferably has a weak hydrophilic surfactant and the; second topsheet layer preferably has a normal hydrophilic surfactant. The total basis weight of a typical material is about 20 to 22 g/m².

Preferably the acquisition/distribution layer 105 comprises carded, resin bonded hiloft nonwoven materials such as, for example, FT-6860, available from Polymer Group, Inc., North America (Landisiville, New Jersey, U.S.A.). They are made of polyethylene terephthalate fibers of 6 dtex, and has a basis weight of about 43 g/m². Alternatively, acquisition/distribution layer 105 comprises chemically treated stiffened cellulosic fiber material, available from Weyerhaeuser Co. (United States) under the trade designation of CMC. In another, preferred embodiment, the acquisition/distribution layer 105 comprises conventional cellulosic fluff material, also known as wood pulp fiber, available from Weyerhaeuser Co. (United States) under the trade name FLINT RIVER:

It should be further understood that the present invention is also applicable to other absorbent articles known commercially by other names, such as incontinent briefs, adult incontinent products, training pants, diaper inserts, facial tissues, paper towels, and the like.

### F. Test Methods

### a. General

All tests are carried out at about 23 +/- 2°C and at about 50 +/- 10% relative humidity.

Unless specified explicitly, the specific synthetic urine used in the test methods is commonly known as JAYCO SYNURINE and is available from Jayco Pharmaceuticals Company of Camp Hill, Pennsylvania. The formula for the synthetic urine is: 20g/l of KCl, 2.0 g/l of Na₂SO₄, 0.85 g/l of (NH₄)H₂PO₄; 0.15 g/l (NH₄)₂HPO₄, 0.19 g/l of CaCl₂, and 0.23, g/l of MgCl₂. All of the chemicals are of reagent grade. The pH of the synthetic urine is in the range of from about 6.0 to about 6.4.

### b. Finished Product Acquisition Test

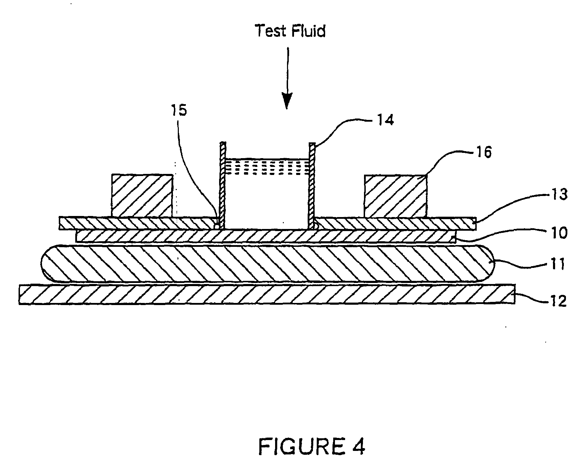

Referring to Figure 4, an absorbent structure 10 is loaded with a 75 ml gush of synthetic urine at a rate of about 15 ml/s using a pump (Model. 7520-00, supplied by Cole Parmer Instruments, Chicago, U.S.A.), from a height of 5 cm above the sample surface. The time to absorb the urine is recorded by a timer. The gush is repeated every 5 minutes at precisely 5 minute gush intervals until the article is sufficiently loaded. Current test data are generated by loading four forms.

The test sample, which comprises an absorbent core and includes a topsheet and a backsheet, is arranged to lie flat on a foam platform 11 within a perspex box (only base 12 of which is shown). A perspex plate 13 having a 5 cm diameter opening substantially in its middle is placed on top of the sample. Synthetic urine is introduced to the sample through a cylinder 14 which is fitted and glued into the opening. Electrodes 15 are located on the lowest surface of the plate, in contact with the surface of the absorbent structure 10. The electrodes are connected to the timer. Loads 16 are placed on top of the plate to simulate, for example, a baby's weight. A pressure of 50 g/cm² is typically utilized for this test.

As test fluid is introduced into the cylinder it typically builds up on top of the absorbent structure thereby completing an electrical circuit between the electrodes. This starts the timer. The timer is stopped when the absorbent structure has absorbed the gush of urine, and the electrical contact between the electrodes is broken.

The acquisition rate is defined as the gush volume absorbed (ml) per unit time (seconds). The acquisition rate is calculated for each gush introduced into the sample. Of particular interest in view of the current invention are the first and the last of the four gushes.

This test is primarily designed to evaluate products having an absorbent capacity of about 300 ml to about 400 ml. If products with significantly different capacities should be evaluated, the settings in particular of the fluid volume per gush should be adjusted appropriately to about 20% of the theoretical capacity, and the deviations should be recorded.

### c. Finished Product Wetness Test

A disposable absorbent article is placed on the apparatus set forth in Figure 4, as discussed under the finished product acquisition test, above, with the following differences. 225 ml of synthetic urine is loaded into the absorbent article sample. The load occurs via 3 gushes, of 75 ml each, at 3 min. intervals. No additional pressure is applied, other than any negligible pressure that may come from the perspex plate.

Following completion of 225 ml of synthetic urine loading, the perspex plate is removed. Two pieces of filter paper (supplied by Hollingsworth & Vose, United Kingdom, of the type ERT FF3.W/S), having dimensions of 12 cm by 12 cm, are weighed, and then placed on the urine loaded diaper. A load of 2 kg over a 10 cm by 10 cm area is applied to the filter paper (*i.e.,* 0.28 psi) for 2 minutes. The filter paper is removed and weighed a second time. The diaper rewet value is defined as the increase in weight (g) of the filter paper.

### d. In Bag Stack Height

Essentially, the in bag stack height is measured by measuring the height of a stack of absorbent products as it is packed into cartons or bags as supplied to the market, and dividing the height by the number of articles in this stack.

It either can be measured by taking one of the bags and carrying out the measurement, or by simulating the pressure of a packed bag in a suitable device (such as a stress-strain measurement device such as provided by INSTRON instruments).

This test has primarily been developed for "bi-folded" products, *i.e*., products which have only one folding line in the cross (width) direction of the article at about the middle part of the article, such that the front and rear part of the article overlay in the bag. For non folded or tri-folded products (with three layers overlying), the results need to be corrected accordingly.

### e. Basis Weight

Basis weights are often referred to for various materials. These can be generated by essentially dividing the weight of a specimen by the area of it. The size of the area as well as the number of rewired replicates depend on the homogeneity of the specimen.

### f. Hydrophilicity / Hydrophobicity

Hydrophilicity (and hence, wetability) are typically defined in terms of contact angle and the surface tension of the fluids and solids involved. This is discussed in detail in, e.g., the American Chemical Society publication entitled "Contact Angle, Wettability and Adhesion", edited by R.F. Gould (copyright 1964). In the context of the current invention, materials can be categorized into three groups:

Materials which are "highly hydrophilic" (abbreviated "h+"). These generally have a contact angle of less than about 80 degrees. Examples include cellulosic fibers and olefinic polymers when they are treated with an effective and strong surfactant (at least when exposed the first time to wetting).

Materials which are "essentially hydrophobic" (abbreviated "h-"): These generally have a contact angle of more than about 100 degrees. Examples include pure olefines (polyethylene/polypropylene) without surfactants (neither at the surface, nor resin incorporated).

Materials which are "moderately hydrophilic" (abbreviated "ho"): These have a contact angle of about 90 degrees. Examples include polypropylene/polyethylene with less effective resin incorporated surfactants, and other less hydrophilic surfactants applied to the surface of such olefins.

### G. Examples

The following examples further describe and demonstrate the preferred embodiments within the scope of the present invention The examples are given solely for the purpose of illustration, and are not to be construed as limitations of the present invention since many variations thereof are possible without departing from its spirit and scope.

### Example 1

This example shows how to manufacture a P-HFAP of the present invention.

4,000 parts of an aqueous solution of 37% acrylic monomer composed of 74.95 mol% of sodium acrylate, 25 mol% of acrylic acid, and 0.05 mol% of N, N'-methylenebis acrylamide is mixed by being stirred with 2.0 parts of sodium persulfate and 0.08 part of 1-ascorbic acid. The mixture of the polymerization system is kept at 60°C and under constant agitation. 20 minutes after the reaction starts, 1.5% of perfume and 2.2% of α-starch carrier is added into the mixture of the polymerization system. 5 minutes after the addition of the perfume and the α-starch carrier, gelation takes place as indicated by the elevation of temperature to over 70°C of the polymerization system. The system is turned to 80°C for another one hour in order to complete the gelation. The hydrated gel polymer is dried with a hot air dryer at 80°C for at least 10 hours, pulverized with a hammer type pulverizing device, and sifted with a 20-mesh mettalic gauze to separate a 20-mesh pass powder as an absorbent polymer having an average particle diameter of about 350 micro meter to obtain P-HFAP.

### Example 2

This example shows how to manufacture a P-HFAP of the present invention.

In this case, the same procedure as in Example 1 is followed, except that the 1.5% of perfume and 2.2% of α-starch carrier are replaced with 0.8gm of baby cologne (code 52338 available, e.g., from Procter & Gamble Inc., Cincinnati, U.S. which is added to 1.2gm of α-starch (available, e.g., from Nippon Starch Inc., Osaka, Japan), and gently blended in a food mixer for 10 minutes.

### Example 3

This example shows how to manufacture a P-HFAP of the present invention. in this case, the same procedure as in Example 1 is followed, except that the 1.5% of perfume and 2.2% of α-starch carrier are replaced with 0.8gm of baby cologne (code 52338 available, e.g., from Procter & Gamble Inc., Cincinnati, US.) which is added to 1.2gm of β-cyclodextrin (available, e.g., from Nihon Shoku-Hin Inc., Tokyo, Japan), and gently blended in a food mixer for 10 minutes.

### Example 4

This example shows how to manufacture a P-HFAP of the present invention. In this case, the same procedure as in Example 2 is followed, except that 17gm of Example-2 P-HFAP is blended with 1000gm of Aquatic CA-L76 (a cross-linked sodium potyacrylate; available from Nippon Shokubai Co. Ltd, Osaka, Japan). The process results in a homogeneous blending of two polymers.

### Example 5

This example shows an absorbent core comprising a P-HFAP which is made by the process of the present invention, for use in a disposable absorbent article.

Wood pulp of Southern Pine is disintegrated and opened in an air flowing chamber. Fibers having an average length of about 3 mm are allowed to fall on a vacuum plate. During the fiber laydown procedure, the P-HFAP of Example 4 is sprinkled into the wood pulp fibers. A core containing 230 to 400 g/m² of wood pulp fibers and 160 to 360 g/m² of the P-HFAP is formed. The core is suitable for use in infant and/or adult incontinent diaper applications. A typical composition of an L-size infant diaper application is as follows:

| Component | Weight (gm) | Percent (wt) |
|---|---|---|
| Wood pulp fiber | 15 | 55.6 |
| P-HFAP | 12 | 44.4 |

### Example 6

This example shows a disposable baby diaper comprising an P-HFAP which is made by the process of the present invention.

The dimensions listed are for a diaper intended for use with a child in the 6-10 kilogram size range. These dimensions can be modified proportionatety for different size children, or for adult incontinence briefs, according to standard practice.
1. Backsheet 0.020-0.038 mm fluid-impermeable film manufactured per U.S. Patent No. 4,687,478; width at top and bottom 33cm; notched inwardly on both sides to a width-at-center of 28.5 cm; length 50.2 cm.
2. Topsheet: carded and thermally bonded staple-length polypropylene fibers (Hercules type 151 polypropylene); width at top and bottom 33 cm; notched inwardly on both sides to a width-at-center of 28.5 cm; length 50.2cm.
3. Absorbent core: comprises 28.6 g of cellulose wood pulp and 4.9 g of the A-HFAP of Example 3; 8.4 mm thick, calendered; width at top and bottom 28.6 cm; notched inwardly at both sides to a width-at-center of 10.2 cm; length 44.5 cm.
4. Elastic leg bands: four individual rubber strips (2 per side); width 4.77 mm; length 370 mm; thickness 0.178 mm (all the foregoing dimensions being in the relaxed state).

The diaper is prepared in standard fashion by positioning the core material covered with the topsheet on the backsheet and gluing.

The elastic bands (designated "inner" and "outer", corresponding to the bands closest to, and farthest from, the core, respectively) are stretched to ca. 50.2 cm and positioned between the topsheet/backsheet along each longitudinal side (2 bands per side) of the core. The inner bands along each side are positioned ca. 55 mm from the narrowest width of the core (measured from the inner edge of the elastic band), this provides a spacing element along each side of the diaper comprising the flexible topsheet/backsheet material between the inner elastic and the curved edge of the core. The inner bands are glued down along their length in the stretched state. The topsheet/backsheet assembly is flexible, and the glued-down bands contract to elasticize the sides of the diaper.

The resulting diaper provides combined fluid adsorption property and properties which (i) do not leak smell or hardly leak smell when the P-HFAP of the diaper is dry, and (ii) which trigger to release perfume easily when the P-HFAP is wet.

### Example 7

This example shows an adult incontinent product comprising an P-HFAP of the present invention.

Following the preparation procedures of Example 4, a heavy weight core is formed having 24 to 40 g of wood pulp fiber and 10 to 20 g of the P-HFAP. The core is subsequently interposed between a fluid-impermeable poly backsheet and a fluid-permeable topsheet to form an adult incontinent product.

### Example 8

This example shows a light weight pantiliner product comprising an P-HFAP of the present invention.

A light weight pantiliner suitable for use between menstrual periods comprises a pad. The pad has a surface area 117 cm²; and contains 3 g of wood pulp fiber and 1.5 g P-HFAP of Example 3. The pad is subsequently interposed between a porous formed-film topsheet according to U.S Patent No. 4,463,045 and a fluid-impermeable backsheet is manufactured per U.S. Patent No. 4,687,478, to form a light weight pantiliner product. The resulting pantiliner provides combined fluid adsorption and antimicrobial properties.

### Example 9

This example shows a sanitary pad comprising an P-HFAP of the present invention.

A catamenial product in the form of a sanitary napkin having two flaps extending outward from its absorbent core is prepared per the design of U.S. Patent No. 4,687,478, Van Tillburg, issued Aug. 18, 1987. However, the pad utilized has a surface are of 117 cm²; and containing 6 to 12 g of wood pulp fiber and 1 to 3 g of the P-HFAP of Example 2. The fluid-permeable topsheet is manufactured per U.S Patent No. 4,463,045. The fluid-impermeable backsheet is manufactured per U.S. Patent No. 4,687,478. The resulting sanitary pad provides combined fluid adsorption and antimicrobial properties.

The aspects and embodiments of the present invention set forth in this document have surprising advantages, including improved efficacy and improved processability. For example, a P-HFAP which is made by the process of the present invention leaks either no perfume smell or only a negligible amount of perfume smell, (which is accepted by consumers) when the P-HFAP is dry. Perfume is not subjected to aging effect and evaporation of perfume during manufacturing, shipment and storage because the dried P-HFAP is very efficient for trapping perfume within HEAP. In addition, the release of perfume is easily triggered by the dynamic swotting force of a wetted polymer. The release of the perfume is generally enhanced by local temperature arise of P-HFAP in a minutes at initial contact with body fluid because the swelling process is an exothermic process.

## Claims

1. A process for making a perfume-impregnated hydrogel-forming absorbent polymer wherein release of a perfume smell is triggered by dynamic swelling force of the polymer when the polymer is wetted, which comprises the steps of: (i) mixing a solid carrier and a perfume with reaction intermidiates of hydrogel-forming absorbent polymer before the gelation point of polymerization of the hydrogel-forming absorbent polymer, (ii) forming the perfume-impregnated hydrogel-forming absorbent polymer, and (iii) drying the perfume-impregnated hydrogel-forming absorbent polymer.

2. The process of claim 1 wherein the mixing step (i) is conducted at a temperature from 50°C to 90°C.

3. The process of claim 1 wherein the perfume is included in the carrier preliminary before addition to the reaction intermediates of hydrogel-forming absorbent polymer.

4. The process of claim 1 wherein the reaction intermediates of hydrogel-forming absorbent polymer comprises linear polymers, oligomers, monomers and a crosslinker.

5. The process of claim 4 wherein the reaction intermediates of hydrogel-forming absorbent polymer further comprises a polymerization initiator and water.

6. The process of claim wherein the solid carrier is glucose.

7. The process of claim 1 wherein the solid carrier is a porous hydrophilic matrix.

8. The process of claim 1 wherein the step of mixing the carrier and the perfume with the reaction intermediates of hydrogel-forming absorbent polymer is conducted from few seconds to 20 minutes before the gelation point of the polymerization.

9. A perfume-impregnated hydrogel-forming absorbent polymer produced by the process of Claim 1.

10. The perfume-impregnated hydrogel-forming absorbent polymer of Claim 9, wherein the ratio of the hydrogel forming absorbent polymer to the perfume is from 100:0.01 to 100:20.

11. A disposable absorbent article comprising the perfume-impregnated hydrogel-forming absorbent polymer of Claim 9.

12. A disposable diaper comprising the perfume-impregnated hydrogel-forming absorbent polymer of claim 7.

13. A disposable absorbent article comprising:
a) a backsheet;
b) a topsheet;
c) an acquisition/distribution layer; and
d) an absorbent core comprising, the perfume-impregnated hydrogel-forming absorbent polymer of claim 9;
wherein the absorbent article has a finished product acquisition rate greater than or equal to 1.9 ml/sec at first gush and greater than or equal to 3.0 ml/sec at fourth gush, and a folded stackheight of less than 9.9mml per pad; and wherein the topsheet retains no more than 0.5g to 1g of fluid as measured by the finished product wetness test.

## Patentansprüche

1. Verfahren zur Herstellung eines Duftstoff-imprägnierten, Hydrogel-bildenden, absorbierenden Polymers, wobei eine Freisetzung eines Duftstoff-Geruchs durch eine dynamische Quellkraft des Polymers ausgelöst wird, wenn das Polymer benetzt wird, welches die Schritte umfasst: (i) Mischen eines festen Trägers und eines Duftstoffs mit einem Reaktions-Zwischenprodukt eines Hydrogel-bildenden absorbierenden Polymers vor dem Gelbildungs-Punkt einer Polymerisation des Hydrogel-bildenden absorbierenden Polymers, (ii) Bilden des Duftstoff-imprägnierten, Hydrogel-bildenden, absorbierenden Polymers, und (iii) Trocknen des Duftstoff-imprägnierten, Hydrogel-bildenden, absorbierenden Polymers.

2. Verfahren nach Anspruch 1, wobei der Misch-Schritt (i) bei einer Temperatur von 50°C bis 90°C durchgeführt wird.

3. Verfahren nach Anspruch 1, wobei der Duftstoff in dem Träger vor der Zugabe des Reaktions-Zwischenproduktes eines Hydrogel-bildenden, absorbierenden Polymers vorläufig enthalten ist.

4. Verfahren nach Anspruch 1, wobei das Reaktions-Zwischenprodukt eines Hydrogel-bildenden, absorbierenden Polymers lineare Polymere, Oligomere, Monomere und ein Vernetzungsmittel umfasst.

5. Verfahren nach Anspruch 4, wobei das Reaktions-Zwischenprodukt eines Hydrogel-bildenden, absorbierenden Polymers ferner einen Polymerisations-Initiator und Wasser umfasst.

6. Verfahren nach Anspruch 1, wobei der feste Träger Glukose ist.

7. Verfahren nach Anspruch 1, wobei der feste Träger eine poröse, hydrophile Matrix ist.

8. Verfahren nach Anspruch 1, wobei der Schritt des Mischens des Trägers und des Duftstoffs mit dem Reaktions-Zwischenprodukt eines Hydrogel-bildenden, absorbierenden Polymers zwischen einigen Sekunden und 20 Minuten vor dem Gelbildungs-Punkt der Polymerisation durchgeführt wird.

9. Duftstoff-imprägniertes, Hydrogel-bildendes, absorbierendes Polymer, das durch das Verfahren nach Anspruch 1 erzeugt ist.

10. Duftstoff-imprägniertes, Hydrogel-bildendes, absorbierendes Polymer nach Anspruch 9, wobei das Verhältnis des Hydrogel-bildenden, absorbierenden Polymers zu dem Duftstoff von 100:0,01 bis 100:20 reicht.

11. Absorbierender Wegwerfartikel umfassend das Duftstoff-imprägnierte, Hydrogel-bildende, absorbierende Polymer nach Anspruch 9.

12. Wegwerfwindel umfassend das Duftstoff-imprägnierte, Hydrogel-bildende, absorbierende Polymer nach Anspruch 7.

13. Absorbierender Wegwerfartikel umfassend:
a) eine Außenlage;
b) eine Decklage;
c) eine Erfassungs-/Verteilungslage; und
d) einen absorbierenden Kern mit dem Duftstoff-imprägnierten, Hydrogel-bildenden, absorbierenden Polymer nach Anspruch 9;
wobei der absorbierende Artikel aufweist eine Erfassungs-Geschwindigkeit des fertigen Produkts von größer als oder gleich 1,9 ml/s bei einem ersten Schwall und von größer als oder gleich 3,0 ml/s bei einem vierten Schwall und eine gefaltete Stapelhöhe von weniger als 9,9 mm pro Kissen; und wobei die Decklage nicht mehr als 0,5 g bis 1 g eines Fluids zurückhält, gemessen durch den Nässetest des fertigen Produkts.

## Revendications

1. Procédé de préparation d'un polymère absorbant formant un hydrogel et imprégné de parfum, dans lequel la libération d'une odeur de parfum est déclenchée par une force de gonflement dynamique du polymère quand le polymère est mouillé, qui comprend les étapes de : (i) mélange d'un support solide et d'un parfum avec les intermédiaires de réaction du polymère absorbant formant un hydrogel, avant le point de gélification de la polymérisation du polymère absorbant formant un hydrogel, (ii) formation du polymère absorbant formant un hydrogel et imprégné de parfum, et (iii) séchage du polymère absorbant formant un hydrogel et imprégné de parfum.

2. Procédé selon la revendication 1, dans lequel l'étape de mélange (i) est mise en oeuvre à une température de 50 à 90°C.

3. Procédé selon la revendication 1, dans lequel le parfum est incorporé dans le support d'une manière préalable avant addition aux intermédiaires de réaction du polymère absorbant formant un hydrogel.

4. Procédé selon la revendication 1, dans lequel les intermédiaires de réaction du polymère absorbant formant un hydrogel comprennent des polymères linéaires, des oligomères, des monomères et un agent de réticulation.

5. Procédé selon la revendication 4, dans lequel les intermédiaires de réaction du polymère absorbant formant un hydrogel comprennent en outre un amorceur de polymérisation et de l'eau.

6. Procédé selon la revendication 1, dans lequel le support solide est le glucose.

7. Procédé selon la revendication 1, dans lequel le support solide est une matrice hydrophile poreuse.

8. Procédé selon la revendication 1, dans lequel l'étape de mélange du support et du parfum avec les intermédiaires de réaction du polymère absorbant formant un hydrogel est mise en oeuvre entre quelques secondes et 20 minutes avant le point de gélification de la polymérisation.

9. Polymère absorbant formant un hydrogel et imprégné de parfum produit par le procédé selon la revendication 1.

10. Polymère absorbant formant un hydrogel et imprégné de parfum selon la revendication 9, dans lequel le rapport du polymère absorbant formant un hydrogel au parfum est de 100:0,01 à 100:20.

11. Article absorbant jetable comprenant le polymère absorbant formant un hydrogel et imprégné de parfum selon la revendication 9.

12. Couche jetable comprenant le polymère absorbant formant un hydrogel et imprégné de parfum selon la revendication 7.

13. Article absorbant jetable comprenant :
a) une feuille de fond ;
b) une feuille de dessus ;
c) une couche de recueil/répartition ; et
d) un coeur absorbant comprenant le polymère absorbant formant un hydrogel et imprégné de parfum selon la revendication 9;
dans lequel l'article absorbant présente une vitesse de recueil du produit fini supérieure ou égale à 1,9 ml/s au premier jet et supérieure ou égale à 3,0 ml/s au quatrième jet, et une hauteur d'empilement après pliage inférieure à 9,9 mm par tampon ; la feuille de dessus ne retenant pas plus de 0,5 g à 1 g de fluide, la mesure étant effectuée par l'essai d'humidité du produit fini.
